# EUROPEAN PATENT SPECIFICATION

(11) **EP 4 688 060 B1**
(45) Date of publication and mention of the grant of the patent: **07.10.2026**
(21) Application number: 24712262.5
(22) Date of filing: 22.03.2024
(51) Int. Cl.: A61M 11/00, A61M 15/00, B05B 15/40, B05B 17/00, B05B 17/06

(54) **HEAD AND/OR NECK-MOUNTED AEROSOL DELIVERY DEVICE**
KOPF- UND/ODER HALSBEFESTIGTE AEROSOLVERABREICHUNGSVORRICHTUNG
DISPOSITIF DE DISTRIBUTION D'AÉROSOL FIXÉ SUR LA TÊTE ET/OU LE COU

(30) Priority: 24.03.2023 EP 23164182
(43) Date of publication of application: 11.02.2026
(73) Proprietor: MED-EL Elektromedizinische Geräte GmbH, 6020 Innsbruck (AT)
(72) Inventor: NASCHBERGER, Raimund, 6020 Innsbruck (AT)
(74) Representative: Konegger, Mario
(86) International application number: PCT/EP2024/057739
(87) International publication number: WO 2024/200247

(56) References cited:
- WO-A1-2021/209637
- CN-A- 111 467 615
- KR-B1- 101 276 911
- US-A- 5 823 434
- US-A1- 2015 034 086
- US-A1- 2018 280 638
- US-A1- 2020 129 709
- US-B1- 11 273 274
- US-B1- 11 433 212

## Description

### FIELD OF THE INVENTION

The present invention is in the field of medical devices. In particular, the invention refers to a head and/or neck-mounted aerosol delivery device configured for delivering bioactive agents to a wearing user by dispensing an aerosol into the respiration area of said user. The invention further refers to a method of priming a corresponding aerosol delivery device.

### BACKGROUND OF THE INVENTION

Respiratory protection devices are worldwide used for preventing users from being exposed to hazardous substances such as toxic gases via inhalation (environmental protection) and/or from exposing others to own-produced hazardous substances such as infectious fluids via exhalation or ejection of respiratory droplets (subjective protection).

US2020129709 discloses a wearable ultrasonic nebulizer-humidifier designed to synchronize aerosol delivery with the user's inhalation. The disclosed headset-style device includes a liquid reservoir, ultrasonic nebulizer, power supply and control logic to align aerosol generation and release with inspiration. This enhances drug delivery efficiency and user comfort, making it suitable for ambulatory respiratory therapy. The design supports hands-free operation, enabling continuous or on-the-go treatment for respiratory conditions.

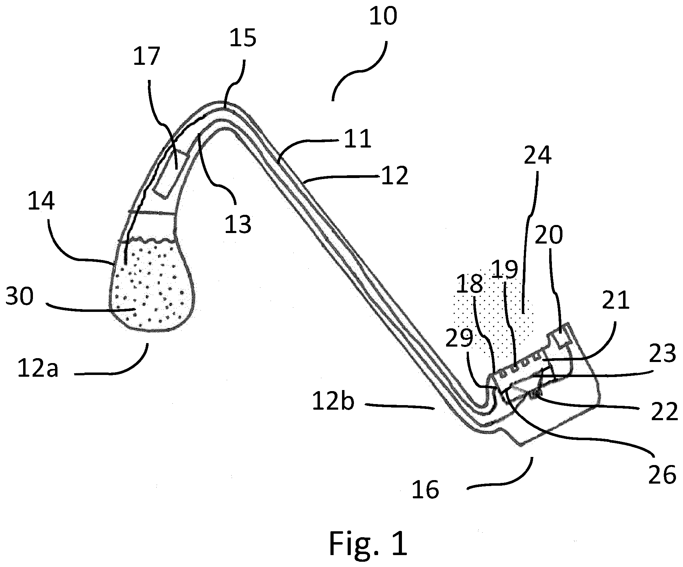

WO2021209637 discloses an aerosol delivery device 10 as known in the prior-art. which is herein re-produced in Fig. 1 as a schematic illustration but modified according to embodiments of the invention. The prior-art aerosol delivery device 10 is described in the following with reference to Fig. 1 only for the elements known in the prior-art. The aerosol delivery device 10 comprises an elongated mount 12 that extends from a fixation end 12a to a dosing end 12b. The fixation end 12a of the elongated mount 12 is configured for being attached to the head and/or neck of a user of the aerosol delivery device 10. The fixation end 12a has a hook-like shape adapted for being attached to the ear of the user by lying on and around the upper part of the ear flap of the patient.

The mount 12 is shaped such that, when the aerosol delivery device 10 is attached to the head and/or neck of the user by the fixation end 12a, the mount 12 extends along the face of the patient and the dosing end 12b is arranged below the fixation end 12a (considered in the vertical direction) and in a proximity of a respiration area 24 located in front of the mouth and nose of the user. When the user inhales, air from the respiration area 24 may enter through the mouth and nose of the user. When the user exhales, air may be expelled from the mouth and nose of the user into the respiration area 24.

The aerosol delivery device 10 further comprises a reservoir 14 configured for storing a liquid bioactive fluid 30, an aerosol generating device 16 arranged at the dosing end 12b and fixedly attached thereto. The aerosol generating device 16 is connected to the reservoir 14 by a connection hose 15 that establishes a fluid connection between the reservoir 14 and the aerosol generating device 16. The aerosol generating device 16 can receive the bioactive fluid 30 stored in the reservoir 14 through the connection hose 15 and is configured for generating an aerosol comprising the bioactive fluid 30 and for generating and dispensing the aerosol into the respiration area 24.

The aerosol delivery device 10 further comprises a control unit 17 that is integrated within the mount 12 and is configured for controlling the aerosol generating device 16. The aerosol generating device 16 dispenses the aerosol into the respiration area 24 when activated by the control unit 17 by means of a corresponding control instruction transmitted through an electric cable 13 by means of which the control unit 17 is connected to the aerosol generating device 16.

The aerosol delivery device 10 further comprises a respiratory activity sensor 20 arranged at the dosing end 12b of the mount 12 and fixedly attached to the aerosol generating device 16. The respiratory activity sensor 20 is configured for detecting respiratory activity in a surrounding environment of the aerosol delivery device 10, for example respiratory activity in the respiration area 24. The respiratory activity sensor 20 is operatively connected to the control unit 17 by means of the electric cable 13. When the respiratory activity sensor 20 detects respiratory activity, a detection signal is sent to the control unit 17 transmitting information about the detected respiratory activity. The control unit 17 is configured for activating the aerosol generating device 16 in response to said detection signal sent by the respiratory activity sensor 20, i.e. when the respiratory activity sensor 20 detects respiratory activity.

The aerosol delivery device 10 in the prior art describes three methods for generating the bioactive aerosol, namely 1) by spraying a fluid with pressure through a spray nozzle, or 2) by spraying a fluid with a vibrating mesh membrane with holes, and 3) by dispensing a dry powder. The first method suffers from high production costs, high energy needs and high noise level due to high pressure levels needed in order to create a dry aerosol that could in the best case only reach the lower airways. Method 3) suffers from high development costs and challenges to provide a reliable and precisely pulsed aerosol from dry powder, which restricts the use only for very high-volume applications.

While mesh nebulisers according to method 2) are widely used and state of the art for handheld applications or respirators, the system is very sensitive to drop formation on the outer side of the membrane and air bubbles within the spray chamber. The tubing may add further undesirable effects and therefore filling, cleaning and storage by the user can be quite cumbersome. The re-filling of the reservoir 14 with bioactive fluid 30 may introduce unwanted particles and/or contaminants, which need countermeasures to ensure reliable and hygienic operations.

### SUMMARY OF THE INVENTION

The present invention aims at solving some of the problems of providing an improved aerosol delivery device overcoming the aforementioned disadvantages of the prior art. According to the invention, there is provided an aerosol delivery device according to claim 1. Preferred embodiments of the invention are defined in the dependent claims.

An aerosol delivery device according to the invention comprises a body-wearable mount adapted for attaching the aerosol delivery device to the head and/or neck of a user, with a reservoir for storing a bioactive fluid and an aerosol generating device. The aerosol generating device comprises a chamber for receiving the bioactive fluid from the reservoir, a membrane with perforation openings and a speaker with a speaker membrane. The speaker membrane may comprise of multiple parts, e.g. a structure and/or an additional sealing element. The membrane is adapted for generating and dispensing from the chamber into a respiration area an aerosol comprising said bioactive fluid through its perforation openings. The chamber of the aerosol generating device is fluidly connectable to the reservoir to receive the bioactive fluid stored in the reservoir. The chamber further comprises two opposing walls, one wall is formed in part by the membrane and the respective opposite wall is formed in part by the speaker membrane. The membrane and speaker membrane are arranged such that only when direct current with pre-defined polarity is applied to the speaker, the speaker membrane is continuously moved away from the perforation openings and thereby allows said bioactive fluid to flow from the chamber to and out through the perforation openings. In a further embodiment the speaker membrane may be coated with a liquid tight material and/or hydrophilic material.

In further embodiments of the invention, the speaker membrane may comprise a suspension that is at least in part structured in its thickness to adjust the stiffness. Without limitation, ablation may be accomplished by e.g. laser ablation or structured in its thickness by stamping or thermoplastic moulding processes. The suspension of the speaker membrane is adapted to not allow fluid flow to and out through the perforation openings for static fluid pressures in the chamber up to at least 5kPa or 10kPa over the environmental pressure. The speaker membrane may further comprise a seal-lip and the perforation openings may be located at a centre of the membrane. The seal-lip may be made from silicone and/or may have a shore hardness of less than 10 Shore A. Alternatively or additionally, the seal-lip may have a ring-shape with an inner diameter surrounding all of the perforation openings in the membrane so that the bioactive fluid cannot flow to the perforation openings when no direct current with pre-defined polarity is applied.

In further embodiments, the shape of the speaker membrane may at least in part conform to the shape of the membrane. The perforation openings may be located at a centre of the membrane and the shape of the speaker membrane may conform to the shape of the membrane in the area of the perforation openings.

In still further embodiments the direct current with pre-determined polarity may have a pre-defined amperage, which may be constant or vary over time. The speaker membrane may be displaceable by at least 0,1mm. The speaker may be a voice-coil type speaker or variable reluctance type speaker and the aerosol delivery device according to embodiments of the invention may comprise a first and/or second check valve.

The present disclosure further refers to a computer-readable storage medium (not claimed) comprising instructions which, when executed by a processor, cause the processor to carry out the method of controlling an aerosol delivery device according to any of the previous described embodiments.

### BRIEF DESCRIPTION OF THE FIGURES

Fig. 1 shows a schematic illustration of an aerosol delivery device according to an embodiment of the invention.
Fig. 2 shows the cross-section view of a schematic illustration of an aerosol generating device of an aerosol delivery device according to embodiments of the invention.
Fig. 3 shows the top view of a schematic illustration of an aerosol generating device of an aerosol delivery device according to embodiments of the invention.
Fig. 4 shows a cross-section view of a schematic illustration of an aerosol generating device of an aerosol delivery device according to embodiments of the invention.
Figs. 5A-C show a schematic illustration of the working principle of an aerosol generating device of an aerosol delivery device according to embodiments of the invention.
Fig. 6 shows the cross-section view of a schematic illustration of an aerosol generating device of an aerosol delivery device according to embodiments of the invention.
Fig. 7 shows a flow diagram schematically illustrating a method (not claimed) of priming an aerosol generating device of an aerosol delivery device according to embodiments of the invention.
Fig. 8 is a timing diagram of an opening and closing sequence synchronized with aerosol generating and dispensing into a respiration area according to embodiments of the disclosure.

### DETAILED DESCRIPTION OF PREFERRED EMBODIMENTS

For the purposes of promoting an understanding of the principles of the invention, reference will now be made to examples illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

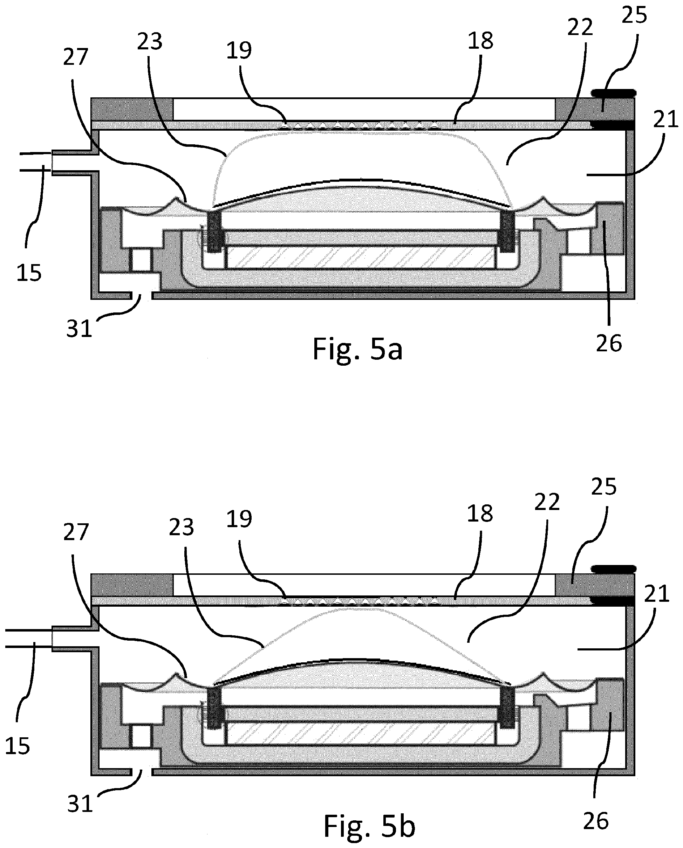

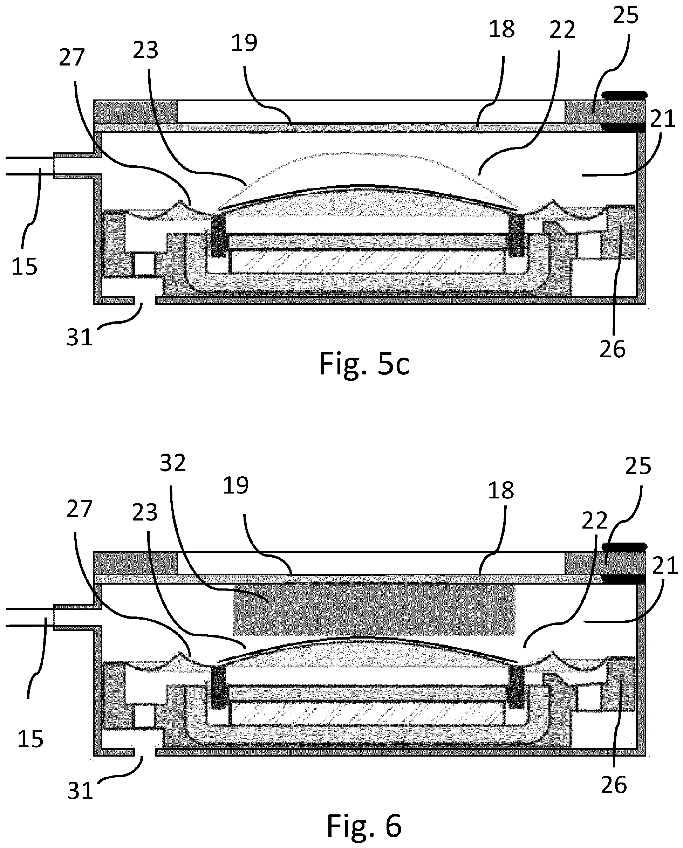

Fig. 1 shows a schematic illustration of an aerosol delivery device 10 according to an embodiment of the invention, with an aerosol generating device 16. The aerosol delivery device 10 according to the present invention comprises a body-wearable mount 12 adapted for attaching the aerosol delivery device 10 to the head and/or neck of a user, a reservoir 14 for storing a bioactive fluid 30, an aerosol generating device 16 comprising a chamber 21 for receiving the bioactive fluid 30 from the reservoir 14, a membrane 18 with perforation openings 19 and a speaker 22 with a speaker membrane 23. The membrane 18 is adapted for generating and dispensing from the chamber 21 into a respiration area 24 an aerosol comprising the bioactive fluid 30 through its perforation openings 19. The chamber 21 of the aerosol generating device 16 is fluidly connectable to the reservoir 14 to receive the bioactive fluid 30 stored in the reservoir 14. The chamber 21 further comprises two opposing walls 25 and 26 and optionally a vent and/or a port to connect a venting element 31 (not shown). One wall, the front wall 25, is formed in part by the membrane 18 and the respective opposite wall, the back wall 26, is formed in part by the speaker membrane 23. The two walls and thus membrane 18 and speaker membrane 23 are arranged such that only when direct current with pre-defined polarity is applied to the speaker 22, the speaker membrane 23 is continuously moved away from the perforation openings 19 and thereby allows said bioactive fluid 30 to flow from the chamber 21 to and out through the perforation openings 19. At rest, i.e. when no direct current is applied to speaker 22, the speaker membrane 23 gently presses against membrane 18 and thereby overlays and closes all of the perforation openings 19 such that no bioactive fluid 30 can flow out through the perforation openings 19. This is described in more detail below with reference to Fig. 5.

The chamber 21 of the aerosol generating device 16 is fluidly connectable to the reservoir 14 with hose 15 and connector 29. Connector 29 may form a mechanically releasable or fixed connection between hose 15 and chamber 21 so that fluid can flow between hose 15 and chamber 21. The releasable connector 29 may comprise a Luer-lock, with a connector part 29a on one end of hose 15 and a mating connector part 29b on a wall of the chamber 21 of the aerosol generating device 16. The reservoir 14 containing the bioactive fluid 30 may be fixated with the respective other end of hose 15, such that bioactive fluid 30 from the reservoir 14 can flow through hose 15 and out from the other, open end. The connector part 29a may comprise a sealing member (not shown) which sealable closes connector part 29a to prevent bioactive fluid 30 to flow out from the reservoir 14 through hose 15 and advantageously protects the bioactive fluid 30 from environmental contaminants when sealed and not in use. Reservoir 14, hose 15 and connector part 29a, if sealed with sealing member, may form a single replaceable unit 33 (not shown).

In one embodiment, mount 12 may comprise a lumen 11 which may be a recess formed within and along the elongate mount 12 with a snap-in opening along the length of the elongate mount 12 from the dosing end 12b to the fixation end 12a. The width of the snap-in opening is smaller than the outer diameter of hose 15, such that hose 15 can be passed through the snap-in opening into the recess by elastically deforming hose 15. The placed hose 15 is guided and hold in place in the lumen 11 through its elastic forces. In another embodiment, mount 12 may comprise one or more fixation clamps along the length of the elongate mount and hose 15 is fixated to mount 12 by means of the one or more fixation clamps. It is however understood by the skilled-in-the-art, that no additional fixation of hose 15 to mount 12 is necessary at all to operate the present invention and the above-described embodiments are entirely optional.

In the context of the invention the term bioactive fluid 30 is understood to be not limited to a fluid including a bioactive agent only, but refers to any fluid, e.g. with or without a pharmaceutically active substance, or a physiological solution, or a fluid comprising odors, vitamins and in general any fluid that has an physical, chemical, bio-chemical or pharmaceutical effect on the organism of the aerosol delivery device 10 wearing user. Further included are fluids comprising marker and/or neutralization substances that may react with the air the user exhales. In case of added marker substances, this can be used for diagnosing purposes and in case of neutralization substances this could be used to neutralize viral loaded exhaled air in an effort to prevent infections of human beings in vicinity of the user. It is also understood, that the before mentioned added substances may be combined in any desirable combination. By way of example only, the fluid my comprise an odor and a substance to neutralize viral load in the exhaled air. Similarly, reference herein to a bioactive agent includes any of the before mentioned added substances or any desirable combinations thereof. Without departing from the invention concept, is readily understood, that the bioactive fluid 30 also includes powders that are ready mixed with fluid by the user prior to use to form a dispensable fluid.

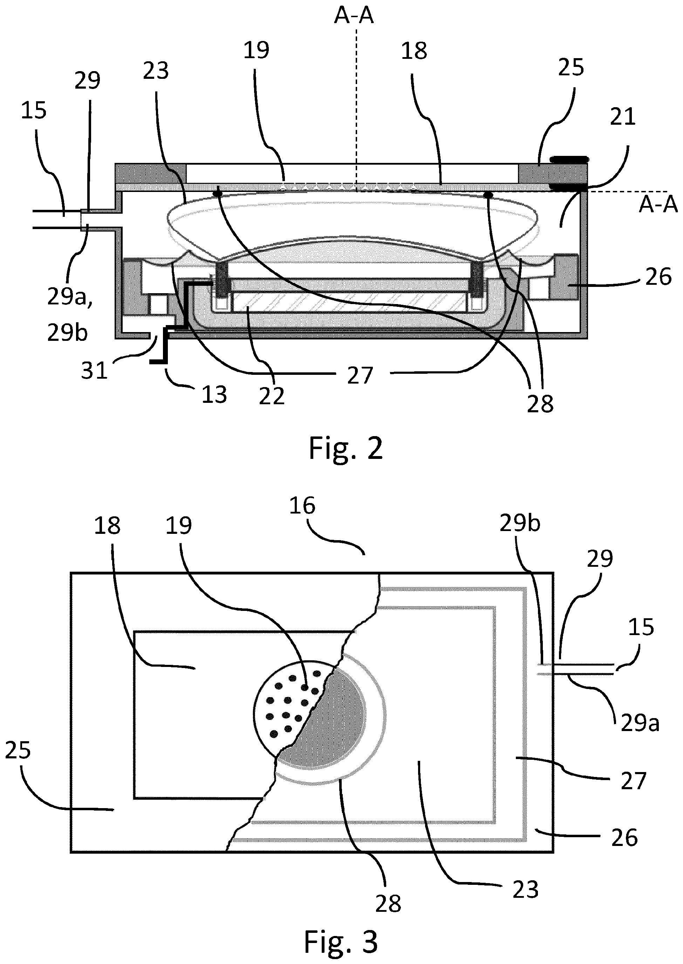

Figure 2 shows the cross-section view of a schematic illustration of an aerosol generating device 16 of an aerosol delivery device in accordance with embodiments of the invention. The aerosol generating device 16 comprising a chamber 21 for receiving the bioactive fluid 30 from the reservoir 14, a membrane 18 with perforation openings 19 and a speaker 22 with a speaker membrane 23. Speaker membrane 23 may comprise of multiple parts and e.g. include a structure. The membrane 18 is adapted for generating and dispensing from the chamber 21 into a respiration area 24 an aerosol comprising said bioactive fluid 30 through its perforation openings 19. The chamber 21 of the aerosol generating device 16 is fluidly connectable by means of connector part 29b to the reservoir 14 to receive the bioactive fluid 30 stored in the reservoir 14. The chamber 21 further comprises two opposing walls 25 and 26, one wall, the front wall 25, is formed in part by the membrane 18 and the respective opposite wall, the back wall 26, is formed in part by the speaker membrane 23. The speaker membrane 23 as shown in Fig. 2 is movably suspended by suspension 27 to allow movement of the speaker membrane 23 toward and away from membrane 18. The suspension 27 is further adapted to hold the speaker membrane 23 at a rest position, i.e. when no direct current is applied to speaker 22, where the speaker membrane 23 is gently pressed against membrane 18 such that all of the perforation openings 19 are overlayed and closed. In other words, at rest, no fluid can flow to and out of the chamber 21 through the perforation openings 19. The suspension 27 may be fabricated with any suitable material and suitable fabrication method, such as e.g. casting. In one embodiment, the suspension 27 may at least in part be laser ablated to adjust the stiffness. In a further embodiment, the suspension 27 may be adapted to not allow fluid flow to and out through the perforation openings 19 for static fluid pressures in the chamber 21 up to at least 5kPa or 10kPa pressure difference to the environmental pressure.

Using an off-the-shelf micro speaker has the advantage, that manufacturing costs of the aerosol delivery device 10 can be considerably reduced. This is because micro speakers are produced in mass production for mass and consumer products, e.g. mobile telephones, headsets and the like. Consequently, production costs are very low for this component. Even though a micro speaker is adapted for generating sound from an applied alternate current, the inventor has found that the micro speaker is still applicable for the above function when operated with direct current. The inventor also found out, that the movement of the speaker membrane 23 of the micro speaker and the forces are sufficient, when a direct current with an amperage and voltage still within the safety-range is applied. Moreover, the power consumption of the speaker is low and allows for prolonged in battery operated device, even with lightweight, small capacity batteries or rechargeable batteries. In one embodiment the speaker membrane 23 is displaceable for at least 0.1mm. The speaker 22 may be a voice-coil type speaker or a variable reluctance type speaker. The inventor found out, that a piezo type speaker would not work, because the displacement of the speaker membrane 23 is more than a magnitude to low.

Direct current in the context of this description does not mean, that the current is kept constant over time, it merely means that the polarity of the current remains the same over time, but the amperage may vary. For example, a current varying over time, but maintaining its polarity is still a direct current. In one advantageous embodiment for the speaker 22 an off-the-shelf micro-speaker is chosen that has a speaker membrane 23 with a liquid tight material. In another advantageous embodiment, the speaker membrane 23 may be coated with a liquid tight material, e.g. silicone. In still another advantageous embodiment the coating may comprise a hydrophilic material.

In one embodiment the perforation openings 19 may be arranged in a centre of membrane 18, as for example shown in figure 2 and figure 3 by the dots. The speaker membrane 23 may comprise a sealing element 28. -Sealing element 28 may be a protrusion integrally formed in speaker membrane 23 and coated with e.g. silicone or be formed out of silicone., Preferably the silicone may have a shore hardness of less than 75 Shore D or 35 Shore D. The sealing element 28 may have a ring-shape with an inner diameter such that the sealing element 28 surrounds all of the perforation openings 19 in the membrane 18. The seal-lip 28 supports the speaker membrane 23 in its rest position to close perforation openings 19 and prevent bioactive fluid 30 flow to and through the perforation openings 19.

Figure 3 shows the top view of a schematic illustration of an aerosol generating device 16 of an aerosol delivery device according to embodiments of the invention. The part to the left of the rupture line AA shows the top view of the device. The part to the right of the rupture line AA shows the top view where the front wall 25 is removed as shown in Fig. 2 with dashed lines A-A so that the back wall 26, of chamber 21, comprising speaker membrane 23, suspension 27 and optional sealing element 28 can be seen. On the left half the front wall 25 is shown, which in part is formed with membrane 18. Membrane 18 comprises perforation openings 19 in its centre. The perforation openings 19 are depicted as small dots and the centre is the area encircled with a solid line. On the right side, the back wall 26 of chamber 21 is visible, which in part is formed by speaker membrane 23. The speaker membrane 23 comprises a suspension 27. In this exemplary depiction suspension 27 is on the circumference of speaker membrane 23. Also shown is a sealing element 28 on the speaker membrane 23 in the embodiment with a ring-shape with an inner diameter surrounding all of the perforation openings 19 in the membrane 18. Chamber 21 further comprises a connector part 29b for interconnection with mating connector part 29a of hose 15, so that bioactive fluid 30 from the reservoir 14 can flow through hose 15 into the chamber 21.

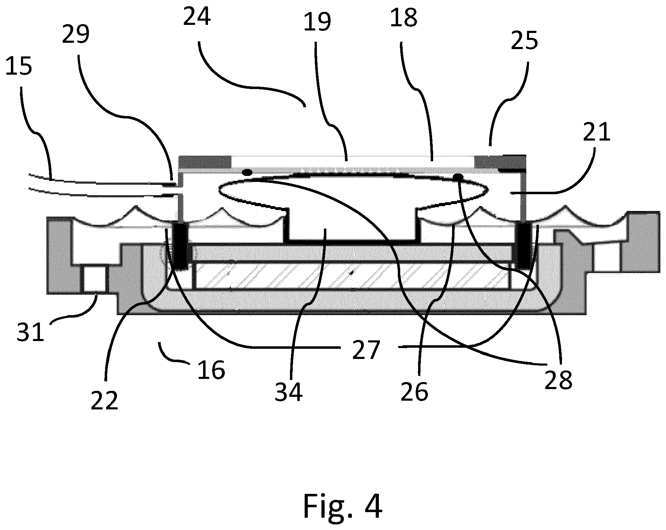

Figure 4 shows the cross-section view of a schematic illustration of an aerosol generating device 16 of an aerosol delivery device according to embodiments of the invention. Compared to figure 2 and figure 3 embodiment, speaker membrane 23 comprises as structure front wall 25, which in part is formed with membrane 18. All other structural parts are substantially the same. As in all the other disclosed embodiments, sealing element 28 may be on the speaker membrane 23. In an alternative embodiment, sealing element 28 may be on elastic structure 34, which is fixedly connected to back wall 26, such that when direct current with pre-defined polarity is applied to the speaker 22, the speaker membrane 23 is continuously moved away from the elastic structure 34 and thereby allows said bioactive fluid 30 to flow from the chamber 21 to and out through the perforation openings 19.

Figures 5A-C show a schematic illustration of the working principle of an aerosol generating device 16 of an aerosol delivery device according to embodiments of the invention. In this example, perforation openings 19 are arranged in the centre of membrane 18. Figure 5A shows the speaker membrane 23 at rest position, i.e. when no direct current is applied to speaker 22. The speaker membrane 23 with or without optional sealing element 28 gently presses against membrane 18 and thereby overlays and closes all of the perforation openings 19 such that no bioactive fluid 30 can flow out through the perforation openings 19. The pressing force is determined by the stiffness of suspension 27 at the per-determined separation distance between membrane 18 and speaker membrane 23. In this example, the shape of the speaker membrane 23 with or without additional structure is adapted to conform to the shape of the membrane 18 in the area of the perforation openings 19. In another example, the shape of the speaker membrane 23 conforms only in part to the shape of the membrane 18. Figure 5B shows the speaker membrane 23 at an interim position between rest (closed) position and open position, where only a relatively small part in the centre of the speaker membrane 23 is pressed against membrane 18. In the interim phase, where the speaker membrane 23 is moved from rest position to open position, the speaker membrane 23 may continuously lift away from membrane 18 starting from the edge towards the centre. This has the advantage, that bioactive fluid 30 in chamber 21 can continuously flow into the continuously changing gap between membrane 18 and speaker membrane 23 and thereby limits air bubbles. If the speaker membrane 23 is moved from the open position into rest (closed) position, the gap between speaker membrane 23 and membrane 18 will be closed starting from the centre of the speaker membrane 23 towards the edge. This has the advantage, that bioactive fluid 30 is pushed out of the gap without trapping bioactive fluid 30 between speaker membrane 23 and membrane 18, which might cause such trapped bioactive fluid 30 unintentionally flow out through perforation openings 19 into the environment. Fig. 5C shows the speaker membrane 23 at an open position. In this position a gap between membrane 18 and speaker membrane 23 exists and bioactive fluid 30 can flow from chamber 21 to and out through perforation openings 19. In this open position, aerosol delivery device 10 may resume its function to deliver an aerosol comprising the bioactive fluid 30 to a respiratory area 24 in a manner known from the prior art.

Any of the embodiments shown in relation to Figures 2 to 5 above may include a squeezable micro-capillary structure 32 comprising micro-capillaries fixated to membrane 18 or speaker membrane 23 in such a manner that it covers the perforation openings 19. A schematic illustration of an aerosol generating device 16 of an aerosol delivery device according to this embodiment of the invention is shown in Figure 6. In the open position, speaker membrane 23 may not squeeze the micro-capillary structure 32 in a way to not let the micro-capillaries collapse and bioactive fluid 30 is able to flow into and out from the micro-capillaries. This in turn allows bioactive fluid 30 to flow out of the chamber 21 into, through and out of the micro-capillaries of micro-capillary structure 32 and through perforation openings 19. In operation aerosol delivery device 10 may generate an aerosol with membrane 18. In the rest position, speaker membrane 23 may squeeze the micro-capillary structure 32 in a way to let the micro-capillaries collapse and no bioactive fluid 30 can flow into, through and out from the micro-capillaries of micro-capillary structure 32. This in turn prevents bioactive fluid 30 to flow out of the chamber 21 into, through and out of the micro-capillaries of micro-capillary structure 32 and through perforation openings 19. One advantage of adding the micro-capillary structure 32 is, that its micro-capillaries act as a filter and retent contaminants. Suitable materials for the micro-capillary structure 32 without limitation and for example are cellulose acetate, cellulose or regenerated cellulose, polytetrafluoroethylene, polyamide, polyether sulfone, cotton and other natural or synthetic polymers. In a further advantageous embodiment, the micro-capillary structure 32 may be optionally made from hydrophilic material or its surface and capillaries may be coated with a hydrophilic material or treated for hydrophilic surface properties.

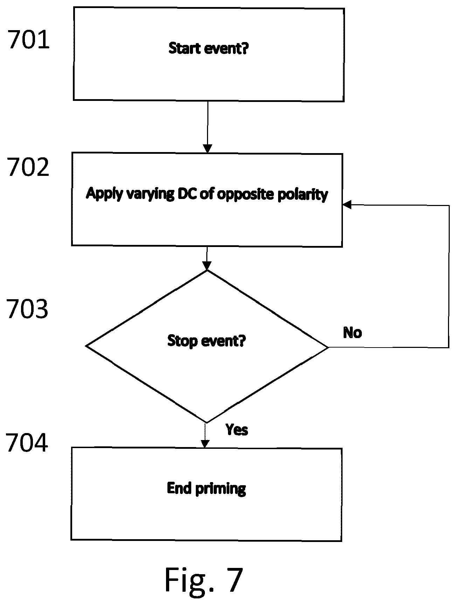

Figure 7 shows a flow diagram schematically illustrating a method (not claimed) of priming an aerosol generating device 16 of an aerosol delivery device according to embodiments of the invention. Priming in the context of this description means a method of filling the fluid carrying elements of the fluid-delivery device such that there almost no air-bubbles at the perforation openings 19 remain. In one embodiment, prior to resume the function to deliver aerosol to a respiratory area 24, it is advantageous to remove any air but fill chamber 21 with bioactive fluid 30. This prevents discontinuous generation and delivery of the aerosol and in the extreme case may not even deliver any aerosol. At step 701, priming starts upon detection of a start event. A start event may be that reservoir 14 by means of hose 15 and connector part 29b is connected to mating connector part 29b to the aerosol generating device 16, thereby establishing a fluid connection so that bioactive fluid 30 can flow through hose 15, connector 29 into chamber 21. Detection of the start event may be accomplished with a sensor, detecting that connection part 29a is connected to connection part 29b. The sensor can be of any known means, including without limitation an electric switch that is connected to control unit 17. Another start event can be a user intervention, where the user manually through a user interface initiates a start event. The user interface may be on the aerosol delivery device 10 or on a remote device (not shown), e.g. through a computing device such as a smart-phone that may connect to the aerosol delivery device 10 wirelessly or via cable. Other ways to initiate priming are equally suitable, without departing from the embodiments of this disclosure. At step 702 in response to detecting a start event, apply and periodically vary the amperage of a direct current with pre-defined polarity to the speaker 22 such that the speaker membrane 23 is continuously pressed against the perforation openings 19 and thereby does not allow said bioactive fluid 30 to flow from the chamber 21 to and out through the perforation openings 19, but periodically changes the volume of the chamber 21 so that bioactive fluid 30 is sucked through hose 15 from the reservoir 14 into the chamber 21 and air is released through a venting element 31 to the environment. At step 703 detect a stop event and if no stop event is detected, continue periodically vary the amperage of direct current in step 702. A stop event can be any of the before mentioned events, i.e. manual through a user or by means of any suitable sensor or expiry of a pre-defined time from step 701. In one example, the sensor may be a current sensor, sensing the supply current to drive speaker 22. Controller 7 can detect changes of the supply current, which occur because displacement of speaker membrane 23 requires more force and thus supply current when chamber 21 is filled with bioactive fluid 30. At step 704 in response to detecting a stop event, stop applying the direct current to the speaker 22. Speaker membrane 23 may resume to the rest (closed) position.

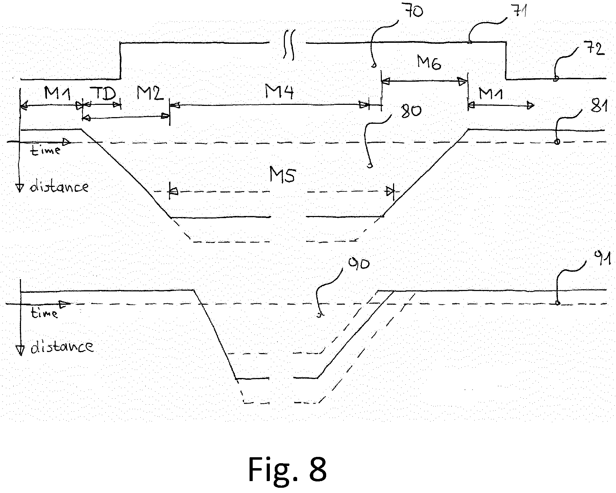

Figure 8 shows a timing diagram of an opening and closing sequence synchronized with aerosol generating and dispensing into the respiration area 24 according to embodiments of the invention. Opening and closing sequence in this respect refers to the abovementioned movement of speaker membrane 23 from rest (close) position to open position and the gradual changes of interim position and gap size during interim phase. The horizontal axis from left to right represents the positive time axis and the vertical axis from top to bottom and perpendicular to the time axis, represents the distance between speaker membrane 23 and membrane 18 as well as the operation status of aerosol generation. The timing diagram can be separated into timing phases M1 through M4. During phase M1, speaker 22 is not supplied with current and speaker membrane 23 is at rest (close) position. In this phase, the aerosol delivery device 10 does not dispense an aerosol to respiratory area 24. During phase M2, direct current of pre-defined polarity is applied to speaker 22 and amperage gradually increased, which continuously displaces speaker membrane 23 so that the distance/gap between speaker membrane 23 and membrane 18 increases. During this phase, bioactive fluid 30 may flow from chamber 21 into the gap between speaker membrane 23 and membrane 18. Typically, the time duration of phase M2 is between 10ms and 100ms. Phase M2 end, when the amperage of the direct current has reached a level, such that speaker membrane 23 has fully lifted from membrane 18 and a minimum distance/gap of at least 0.1mm, preferably 0.2mm, but less than 0,4mm between speaker membrane 23 and membrane 18 is reached. This corresponds to the above-mentioned open position. During phase M4, the direct current is kept at a constant amperage or alternatively is varied over time for as long as aerosol delivery to the respiration area 24 is desired. If aerosol delivery shall be stopped, phase M3 is initiated. During phase M2, the direct current applied to speaker 22 is gradually decreased so that the distance between speaker membrane 23 and membrane 18 decreases. Phase M3 ends, when no direct current is applied to speaker 22 and speaker membrane 23 has reached rest (closed) position.

Aerosol generation and delivery with aerosol generation device 16 can start in phase M2. Preferably aerosol generation is started after a certain pre-defined time-delay TD has lapsed after starting phase M2. This is shown in figure 7 with the status changing from Off-state 60 to On-state 61. The aerosol is generated in the way known from the prior art by applying an alternate current to membrane 18, which typically is a piezo membrane or any other known vibrating actuator, so as to cause membrane 18 with perforation openings 19 to vibrate. This causes bioactive fluid 30 within the capillary of perforation openings 19 to be expelled into respiration area 24 and form an aerosol comprising the bioactive fluid 30 and on the other hand suck bioactive fluid 30 into the capillary of perforation openings 19.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

### LIST OF ABBREVIATIONS

aerosol delivery device 10
lumen 11
mount 12
fixation end 12a
dosing end 12b
electric cable 13
reservoir 14
hose 15
aerosol generating device 16
control unit 17
membrane 18
perforation openings 19
respiratory activity sensor 20
chamber 21
speaker 22
speaker membrane 23
respiration area 24
front wall 25
back wall 26
suspension 27
sealing element 28
connector 29
connector part 29a
connector part 29b
bioactive fluid 30
venting element 31
micro-capillary structure 32
replaceable unit 33
elastic structure 34

## Claims

1. An aerosol delivery device (10) comprising:
a body-wearable mount (12) adapted for attaching the aerosol delivery device (10) to the head and/or neck of a user,
a reservoir (14) for storing a bioactive fluid (30),
an aerosol generating device (16) comprising a chamber (21) for receiving the bioactive fluid (30) from the reservoir (14), a membrane (19) with perforation openings (19a) and a speaker (22) with a speaker membrane (23),
wherein the membrane (19) is adapted for generating and dispensing from the chamber (21) into a respiration area (24) an aerosol comprising said bioactive fluid (30) through its perforation openings (19a),
wherein the chamber (21) of the aerosol generating device (16) is fluidly connectable to the reservoir (14) to receive the bioactive fluid (30) stored in the reservoir (14),
wherein the chamber (21) further comprises two opposing walls (25, 26), one wall (25) is formed in part by the membrane (19) and the respective opposite wall (26) is formed in part by the speaker membrane (23), and
wherein the membrane (19) and speaker membrane (23) are arranged such that only when direct current with pre-defined polarity is applied to the speaker (22), the speaker membrane (23) is continuously moved away from the perforation openings (19a) and thereby allows said bioactive fluid (30) to flow from the chamber (21) to and out through the perforation openings (19a).

2. The aerosol delivery device (10) of claim 1, wherein the speaker membrane (23) is coated with a liquid tight material.

3. The aerosol delivery device (10) of claim 2, wherein the speaker membrane (23) coating is hydrophilic.

4. The aerosol delivery device (10) of claim 1, wherein the speaker membrane (23) comprises a suspension (27) that is at least in part laser ablated to adjust the stiffness.

5. The aerosol delivery device (10) of claim 4, wherein the suspension (27) of the speaker membrane (23) is adapted to not allow fluid flow to and out through the perforation openings (19a) for static fluid pressures in the chamber (21) up to at least 5kPa or 10kPa over the environmental pressure.

6. The aerosol delivery device (10) of any of claims 1 to 5, wherein the speaker membrane (23) comprises a sealing element (28) and the perforation openings (19a) are located at a centre of the membrane (19).

7. The aerosol delivery device (10) of claim 6, wherein the sealing element (28) is made from silicone.

8. The aerosol delivery device (10) of claim 7, wherein the silicone has a shore hardness of less than 10 Shore A.

9. The aerosol delivery device (10) of any of claims 6 to 8, wherein the sealing element (28) has a ring-shape with an inner diameter surrounding all of the perforation openings (19a) in the membrane (19) so that the bioactive fluid (30) cannot flow to the perforation openings (19a) when no direct current with pre-defined polarity is applied.

10. The aerosol delivery device (10) of any of the preceding claims, wherein the shape of the speaker membrane (23) at least in part conforms to the shape of the membrane (19).

11. The aerosol delivery device (10) of claim 10, wherein the perforation openings (19a) are located at a centre of the membrane (19) and the shape of the speaker membrane (22) conforms to the shape of the membrane (19) in the area of the perforation openings (19a).

12. The aerosol delivery device (10) of any of the preceding claims, wherein the direct current with pre-determined polarity has a pre-defined amperage.

13. The aerosol delivery device (10) of any of the preceding claims, wherein the direct current with pre-determined polarity has a constant or varying amperage over time.

14. The aerosol delivery device (10) of any of the preceding claims, wherein the speaker membrane (23) is displaceable by at least 0.1mm.

15. The aerosol delivery device (10) of claim 14, wherein the speaker (22) is a voice-coil type speaker or variable reluctance type speaker.

## Patentansprüche

1. Aerosolabgabevorrichtung (10), umfassend:
eine am Körper tragbare Halterung (12), die dazu ausgebildet ist, die Aerosolabgabevorrichtung (10) am Kopf und/oder Hals eines Benutzers zu befestigen, ein Reservoir (14) zur Speicherung einer bioaktiven Flüssigkeit (30), eine Aerosolerzeugungsvorrichtung (16) mit einer Kammer (21) zur Aufnahme der bioaktiven Flüssigkeit (30) aus dem Reservoir (14), einer Membran (19) mit Perforationsöffnungen (19a) und einem Lautsprecher (22) mit einer Lautsprechermembran (23),
wobei die Membran (19) dazu ausgebildet ist, aus der Kammer (21) durch ihre Perforationsöffnungen (19a) ein die bioaktive Flüssigkeit (30) umfassendes Aerosol in einen Atembereich (24) zu erzeugen und abzugeben,
wobei die Kammer (21) der Aerosolerzeugungsvorrichtung (16) mit dem Reservoir (14) strömungsverbunden werden kann, um die im Reservoir (14) gespeicherte bioaktive Flüssigkeit (30) aufzunehmen,
wobei die Kammer (21) weiterhin zwei gegenüberliegende Wände (25, 26) aufweist, wobei eine Wand (25) teilweise von der Membran (19) gebildet wird und die gegenüberliegende Wand (26) teilweise von der Lautsprechermembran (23) gebildet wird, und
wobei die Membran (19) und die Lautsprechermembran (23) derart angeordnet sind, dass die Lautsprechermembran (23) nur dann kontinuierlich von den Perforationsöffnungen (19a) weg bewegt wird, wenn Gleichstrom mit vorgegebener Polarität an den Lautsprecher (22) angelegt wird, und dadurch der bioaktiven Flüssigkeit (30) ermöglicht wird, von der Kammer (21) zu den Perforationsöffnungen (19a) und durch diese hindurch auszuströmen.

2. Aerosolabgabevorrichtung (10) nach Anspruch 1, wobei die Lautsprechermembran (23) mit einem flüssigkeitsdichten Material beschichtet ist.

3. Aerosolabgabevorrichtung (10) nach Anspruch 2, wobei die Beschichtung der Lautsprechermembran (23) hydrophil ist.

4. Aerosolabgabevorrichtung (10) nach Anspruch 1, wobei die Lautsprechermembran (23) eine Aufhängung (27) umfasst, die zumindest teilweise laserabgetragen ist, um die Steifigkeit einzustellen.

5. Aerosolabgabevorrichtung (10) nach Anspruch 4, wobei die Aufhängung (27) der Lautsprechermembran (23) dazu ausgebildet ist, bei statischen Flüssigkeitsdrücken in der Kammer von bis zu mindestens 5 kPa oder 10 kPa über dem Umgebungsdruck keinen Flüssigkeitsstrom zu den Perforationsöffnungen (19a) und durch diese hindurch zuzulassen.

6. Aerosolabgabevorrichtung (10) nach einem der Ansprüche 1 bis 5, wobei die Lautsprechermembran (23) ein Dichtungselement (28) umfasst und die Perforationsöffnungen (19a) im Zentrum der Membran (19) angeordnet sind.

7. Aerosolabgabevorrichtung (10) nach Anspruch 6, wobei das Dichtungselement (28) aus Silikon besteht.

8. Aerosolabgabevorrichtung (10) nach Anspruch 7, wobei das Silikon eine Shore-Härte von weniger als 10 Shore A aufweist.

9. Aerosolabgabevorrichtung (10) nach einem der Ansprüche 6 bis 8, wobei das Dichtungselement (28) ringförmig ausgebildet ist und einen Innendurchmesser aufweist, der alle Perforationsöffnungen (19a) in der Membran (19) umgibt, sodass die bioaktive Flüssigkeit (30) nicht zu den Perforationsöffnungen (19a) strömen kann, wenn kein Gleichstrom mit vorgegebener Polarität angelegt wird.

10. Aerosolabgabevorrichtung (10) nach einem der vorhergehenden Ansprüche, wobei die Form der Lautsprechermembran (23) zumindest teilweise der Form der Membran (19) entspricht.

11. Aerosolabgabevorrichtung (10) nach Anspruch 10, wobei die Perforationsöffnungen (19a) im Zentrum der Membran (19) angeordnet sind und die Form der Lautsprechermembran (23) der Form der Membran (19) im Bereich der Perforationsöffnungen (19a) entspricht.

12. Aerosolabgabevorrichtung (10) nach einem der vorhergehenden Ansprüche, wobei der Gleichstrom mit vorgegebener Polarität eine vorgegebene Stromstärke aufweist.

13. Aerosolabgabevorrichtung (10) nach einem der vorhergehenden Ansprüche, wobei der Gleichstrom mit vorgegebener Polarität über die Zeit eine konstante oder variable Stromstärke aufweist.

14. Aerosolabgabevorrichtung (10) nach einem der vorhergehenden Ansprüche, wobei die Lautsprechermembran (23) um mindestens 0,1 mm verschiebbar ist.

15. Aerosolabgabevorrichtung (10) nach Anspruch 14, wobei der Lautsprecher (22) ein Schwingspulenlautsprecher oder ein Lautsprecher mit variabler Reluktanz ist.

## Revendications

1. Dispositif de délivrance d'aérosol (10) comprenant :
un support portable sur le corps (12) adapté pour fixer le dispositif de délivrance d'aérosol (10) à la tête et/ou au cou d'un utilisateur, un réservoir (14) destiné à stocker un fluide bioactif (30), un dispositif de génération d'aérosol (16) comprenant une chambre (21) destinée à recevoir le fluide bioactif (30) provenant du réservoir (14), une membrane (19) comportant des ouvertures de perforation (19a) et un haut-parleur (22) comprenant une membrane de haut-parleur (23),
dans lequel la membrane (19) est adaptée pour générer et délivrer depuis la chambre (21) vers une zone respiratoire (24), à travers ses ouvertures de perforation (19a), un aérosol comprenant ledit fluide bioactif (30),
dans lequel la chambre (21) du dispositif de génération d'aérosol (16) peut être reliée fluidiquement au réservoir (14) afin de recevoir le fluide bioactif (30) stocké dans le réservoir (14),
dans lequel la chambre (21) comprend en outre deux parois opposées (25, 26), une paroi (25) étant formée en partie par la membrane (19) et la paroi opposée correspondante (26) étant formée en partie par la membrane de haut-parleur (23), et
dans lequel la membrane (19) et la membrane de haut-parleur (23) sont agencées de telle sorte que la membrane de haut-parleur (23) n'est déplacée de manière continue à l'écart des ouvertures de perforation (19a) que lorsqu'un courant continu de polarité prédéfinie est appliqué au haut-parleur (22), permettant ainsi audit fluide bioactif (30) de s'écouler depuis la chambre (21) vers les ouvertures de perforation (19a) et à travers celles-ci.

2. Dispositif de délivrance d'aérosol (10) selon la revendication 1, dans lequel la membrane de haut-parleur (23) est revêtue d'un matériau étanche aux liquides.

3. Dispositif de délivrance d'aérosol (10) selon la revendication 2, dans lequel le revêtement de la membrane de haut-parleur (23) est hydrophile.

4. Dispositif de délivrance d'aérosol (10) selon la revendication 1, dans lequel la membrane de haut-parleur (23) comprend une suspension (27) qui est au moins partiellement ablatée au laser afin d'ajuster sa rigidité.

5. Dispositif de délivrance d'aérosol (10) selon la revendication 4, dans lequel la suspension (27) de la membrane de haut-parleur (23) est conçue de manière à ne pas permettre un écoulement du fluide vers et à travers les ouvertures de perforation (19a) pour des pressions statiques du fluide dans la chambre allant jusqu'à au moins 5 kPa ou 10 kPa au-dessus de la pression ambiante.

6. Dispositif de délivrance d'aérosol (10) selon l'une quelconque des revendications 1 à 5, dans lequel la membrane de haut-parleur (23) comprend un élément d'étanchéité (28) et les ouvertures de perforation (19a) sont situées au centre de la membrane (19).

7. Dispositif de délivrance d'aérosol (10) selon la revendication 6, dans lequel l'élément d'étanchéité (28) est réalisé en silicone.

8. Dispositif de délivrance d'aérosol (10) selon la revendication 7, dans lequel le silicone présente une dureté inférieure à 10 Shore A.

9. Dispositif de délivrance d'aérosol (10) selon l'une quelconque des revendications 6 à 8, dans lequel l'élément d'étanchéité (28) présente une forme annulaire avec un diamètre intérieur entourant l'ensemble des ouvertures de perforation (19a) de la membrane (19), de sorte que le fluide bioactif (30) ne puisse pas s'écouler vers les ouvertures de perforation (19a) lorsqu'aucun courant continu de polarité prédéfinie n'est appliqué.

10. Dispositif de délivrance d'aérosol (10) selon l'une quelconque des revendications précédentes, dans lequel la forme de la membrane de haut-parleur (23) correspond au moins en partie à la forme de la membrane (19).

11. Dispositif de délivrance d'aérosol (10) selon la revendication 10, dans lequel les ouvertures de perforation (19a) sont situées au centre de la membrane (19) et la forme de la membrane de haut-parleur (23) correspond à la forme de la membrane (19) dans la zone des ouvertures de perforation (19a).

12. Dispositif de délivrance d'aérosol (10) selon l'une quelconque des revendications précédentes, dans lequel le courant continu de polarité prédéfinie présente une intensité prédéfinie.

13. Dispositif de délivrance d'aérosol (10) selon l'une quelconque des revendications précédentes, dans lequel le courant continu de polarité prédéfinie présente une intensité constante ou variable au cours du temps.

14. Dispositif de délivrance d'aérosol (10) selon l'une quelconque des revendications précédentes, dans lequel la membrane de haut-parleur (23) peut être déplacée d'au moins 0,1 mm.

15. Dispositif de délivrance d'aérosol (10) selon la revendication 14, dans lequel le haut-parleur (22) est un haut-parleur à bobine mobile ou un haut-parleur à réluctance variable.
